(19) Europäisches Patentamt
European Patent Office
Office européen des brevets

(11) Numéro de publication: **0 005 654**
**B1**

(12) **FASCICULE DE BREVET EUROPEEN**

(45) Date de publication du fascicule de brevet: **25.11.81**

(21) Numéro de dépôt: **79400167.7**

(22) Date de dépôt: **14.03.79**

(51) Int. Cl.³: **C 07 D 211/12,**
C 07 D 401/06,
C 07 D 211/34,
C 07 D 211/22

(54) **Procédé d'isomérisation des dérivés de la vinyl-3 pipéridine.**

(30) Priorité: **23.03.78 FR 7808449**
**09.02.79 FR 7903291**

(43) Date de publication de la demande:
**28.11.79 Bulletin 79/24**

(45) Mention de la délivrance du brevet:
**25.11.81 Bulletin 81/47**

(84) Etats Contractants Désignés:
**BE CH DE FR GB IT LU NL SE**

(56) Documents cités:
**FR - A - 2 354 771**

**BULLETIN DE LA SOCIETE CHIMIQUE DE FRANCE, fascicule n° 2, février 1972, Paris P. MOREAU, A. CASADEVALL et E. CASADEVALL, "Mécanisme d'épimérisation des cétones cycliques $\alpha$-halogènes en milieu acide", pages 649 à 656**

(73) Titulaire: **PHARMINDUSTRIE**
**35 Quai du Moulin de Cage**
**F-92231 Gennevilliers (FR)**

(72) Inventeur: **Barieux, Jean-Jacques**
**18, rue Tonkin**
**F-69100 Villeurbanne (FR)**
Inventeur: **Dubroeucq, Marie-Christine**
**1, rue de la Sourde**
**F-95170 Deuil-La-Barre (FR)**
Inventeur: **Rocquet, François**
**103 bis, rue de Paris**
**F-95207 Viarmes (FR)**

(74) Mandataire: **Houssin, Jean Produits Chimiques Ugine Kuhlmann et al,**
**Service Propriété Industrielle Tour Manhattan**
**Cedex 21**
**F-92087 Paris la Défense (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 005 654 B1

Procédé d'isomérisation des dérivés de la vinyl-3 pipéridine

La présente invention a pour objet un procédé d'isomérisation des dérivés de la vinyl-3 pipéridine, plus précisément un procédé d'épimérisation du groupe vinyle en position 3 de ces dérivés.

Ce procédé permet de transformer un composé répondant à la formule générale:

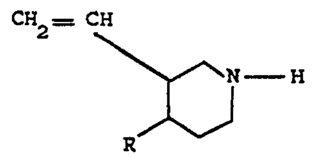

(I)

dans laquelle R est un atome d'hydrogène, un groupe alkyle ayant 1 à 4 atomes de carbone, un groupe phényle non substitué ou substitué par un atome de chlore, de brome ou de fluor ou par un groupe alkyle ayant 1 à 4 atomes de carbone, alcoxy ayant 1 à 4 atomes de carbone ou trifluorométhyle, ou le groupe — $(CH_2)_n$ — X — Z dans lequel n est 1 ou 2, X est un groupe méthylène ou carbonyle et Z représente un groupe alcoxy ayant 1 à 4 atomes de carbone, un reste aromatique ayant 6 à 10 atomes de carbone, éventuellement substitué par un atome de chlore, de brome ou de fluor ou par un groupe alkyle ayant 1 à 4 atomes de carbone, alcoxy ayant 1 à 4 atomes de carbone ou trifluorométhyle, en particulier le groupe phényle, ou un reste quinolyle-4 ou indolyle-2, éventuellement substitué par un atome de chlore, de brome ou de fluor ou par un groupe alkyle ayant 1 à 4 atomes de carbone ou alcoxy ayant 1 à 4 atomes de carbone, et pour lequel le carbone porteur du groupe vinyle possède la configuration rectus (R en abrégé) en le composé correspondant de formule (I) pour lequel le carbone porteur du groupe vinyle possède la configuration sinister (S en abrégé), et vice versa.

La réaction peut être schématisée comme suit:

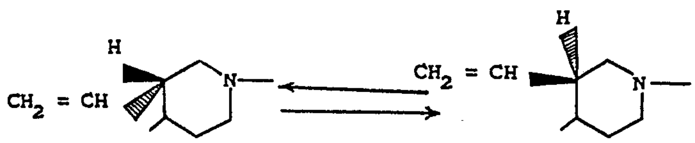

Le procédé selon l'invention est en particulier applicable aux produits, dérivés des alcaloïdes du quinquina, répondant à la formule:

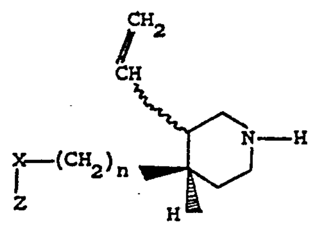

(II)

dans laquelle n, X et Z ont les significations indiquées à la formule (I), et en particulier ceux pour lesquels Z est un reste quinolyle-4 ou indolyle-2, non substitué ou substitué par un atome de chlore, de brome ou de fluor ou par un groupe alkyle ayant 1 à 4 atomes de carbone ou alcoxy ayant 1 à 4 atomes de carbone.

Le procédé selon l'invention consiste à chauffer, au sein d'un solvant protique ou d'un mélange de solvants protiques, en présence de formaldéhyde et à une température supérieure à 50°C et inférieure ou égale à 160°C ou en l'absence de formaldéhyde et à une température située dans la zone 120°C—160°C, un composé de formule (I) pour lequel le carbone porteur du groupe vinyle a la configuration rectus ou sinister, partiellement ou totalement salifié. On isole et purifie ensuite le composé de formule (I) pour lequel le carbone porteur du groupe vinyle possède la configuration sinister ou rectus ainsi formé par des méthodes classiques, physiques (chromatographie, etc...) ou chimiques (formation de sel et régénération de la base libre, etc...).

Comme exemples de solvants protiques on peut citer l'eau et les alcools, en particulier le méthanol et l'éthanol.

Quand le procédé est exécuté en présence de formol, les quantités de formol utilisées vont de préférence de 0,1 mole à 3 moles de formol pour une mole de composé de formula (I) à transformer. Mais il est également possible d'employer des quantités de formol en dehors de ce domaine.

Un mode opératoire particulièrement avantageux consiste à chauffer le composé de formule (I) à une température comprise dans la zone 120°C—160°C, dans un milieu aqueux ou dans un milieu mixte eau + alcool, dont le pH est inférieur à 9 et en particulier est entre 1 et 4, en l'absence de formaldéhyde. Un autre mode opératoire avantageux, particulièrement utile dans le cas des produits

facilement dégradés par la chaleur, consiste à chauffer le composé de formule (I) à une température comprise dans la zone 50°C—80°C, dans un milieu aqueux ou dans un milieu mixte eau + alcool dont le pH est inférieur à 9, en présence de formaldéhyde. Ces manipulations peuvent être réalisées par exemple dans un autoclave ou un tube scellé. Comme milieux mixtes eau + alcool utilisables on peut citer en particulier les milieux eau + éthanol.

Le procédé selon l'invention permet de préparer de manière simple, à partir de composés optiquement actifs existant à l'état naturel ou facilement accessibles à partir de produits naturels, des composés optiquement actifs qui n'existent pas à l'état naturel et dont la synthèse serait longue et délicate.

Les composés obtenus par le procédé selon l'invention sont suceptibles d'avoir des applications thérapeutiques. Ils peuvent aussi être utilisés comme produits intermédiaires pour la synthèse de composés thérapeutiquement utiles, en particulier pour la synthèse, selon des procédés classiques, des composés doués d'activités antiarythmique et antimalarique décrits dans le brevet français n° 2 012 152 et dans les brevets U.S. 3 753 992 et 3 857 846.

La structure des composés obtenus par la procédé selon l'invention a été déterminée en particulier par leur spectre de résonance magnétique nucléaire (R.M.N. en abrégé). Ce spectre permet, par la position des pics correspondant aux protons numérotés 10, 11 et 11' dans la formule (III) cidessous, d'identifier la configuration (sinister ou rectus) du carbone porteur du groupe vinyle (carbone numéroté 3):

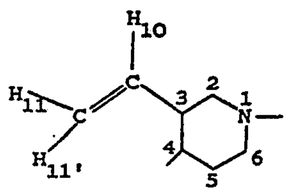

(III)

Les exemples suivants illustrent l'invention sans la limiter.

Exemple 1

*Préparation de la (méthoxy-6 quinolyl-4)-1 [vinyl-3 (S) pipéridyl-4(R)]-3 propanone-1.*

A 2,1 g de (méthoxy-6 quinolyl-4)-1[vinyl-3(R) pipéridyl-4 (R)]-3- propanone-1 (quinicine) on ajoute 20 ml d'eau distillée et on amène le pH à 3,5 par addition d'une solution N d'acide sulfurique. On introduit ce mélange dans un autoclave en acier inoxydable de 225 ml et on chauffe 48 h à 140°C. On alcalinise ensuite la solution par addition d'une solution 2N d'hydroxyde de sodium et on extrait avec de l'éther. L'extrait éthéré est lavé avec de l'eau, séché sur du sulfate de sodium anhydre et évaporé à sec.

Le résidu obtenu (1,7 g) est dissous dans un peu d'un mélange toluène-diéthylamine 9/1' et fixé sur une colonne contenant 500 g de silice. On élue ensuite avec un mélange toluènediéthylamine 9/1, sous une pression de 4 bars. On isole ainsi 0,51 g du produit de départ (quinicine) et 1,08 g de (méthooxy-6 quinolyl-4)-1[vinyl-3(S) pipéridyl-4(R)]-3 propanone-1. Ce dernier composé est mis en solution dans le méthanol et transformé en chlorhydrate par addition d'une solution 8N d'acide chlorhydrique dans le méthanol.

Caractéristiques du chlorhydrate de (méthoxy-6 quinolyl-4)-1[vinyl-3(S) pipéridyl-4(R)]-3 propanone-1:

—point de fusion: 171°C

—pouvoir rotatoire (mesuré dans l'eau à 25°C):

$$[\alpha]^{25}_{D} = -33,3°$$

—spectre de R.M.N. (solvant: deutérochloroforme; référence: tétraméthylsilane):

$$\delta_{10} : 5,6 \text{ ppm}$$

$$\delta_{11,\ 11'} : 5,1 \text{ ppm}$$

La quinicine, produit départ, peut être preparée comme indiqué par HESSE[Ann. 178 (1875), 244—266].

Exemple 2

*Préparation de la (quinolyl-4)-1[vinyl-3(S) pipéridyl-4(R)]-3 propanone-1*

11,3 g d'oxalate de (quinolyl-4)-1[vinyl-3(R) pipéridyl-4(R)]-3 propanone-1 (oxalate de cinchonicine) sont dissous dans 110 ml d'eau permutée. La solution ainsi obtenue est amenée à pH 3,4 par addition d'une solution 5N d'acide chlorhydrique. On introduit cette solution dans un autoclave en acier inoxydable de 225 ml et on chauffe 48 h à 140°C. On alcalinise ensuite la solution par addition

d'une solution 2N d'hydroxyde de sodium et on extrait avec de l'éther. L'extrait éthéré est lavé à l'eau, séché sur du sulfate de sodium anhydre, et évaporé à sec.

Le résidu obtenu est dissous dans un peu d'un mélange toluène-diéthylamine 9/1 et fixé sur une colonne contenant 500 g de silice. On élue ensuite avec un mélange toluène-diéthylamine 9/1, sous une pression de 4 bars. On isole ainsi 2 g de (quinolyl-4)-1[vinyl-3(S) pipéridyl-4(R)]-3 propanone-1 sous forme d'une huile. Cette huile est mise en solution dans l'acétone et le produit ci-dessus est transformé en son oxalate par addition d'une solution 15M d'acide oxalique dans l'acétone. L'oxalate est hygroscopique.

Caractéristiques de l'oxalate de (quinolyl-4)-1[vinyl-3(S) pipéridyl-4(R)]-3 propanone-1:

—spectre de R.M.N. (solvant: deutérochloroforme; référence: tétraméthylsilane):

$$\delta_{10} : 5{,}4 \text{ ppm}$$

$$\delta_{11,\ 11'} : 5 \text{ ppm}$$

La cinchonicine, produit de départ, peut être préparée comme indiqué par HESSE[Ann., 178 (1875), 244—266].

Exemple 3

*Préparation de la méthoxy-6{[vinyl-3(S) pipéridyl-4(R)]-3 propyl-1}-4 quinoléine*

2,1 g de méthoxy-6{[vinyl-3(R) pipéridyl-4(R)]-3 propyl-1}-4 quinoléine sont dissous dans 20 ml d'eau distillée. On ajuste le pH à 2 par addition d'une solution 5N d'acide sulfurique. On introduit ce mélange dans un autoclave en acier inoxydable de 225 ml et on chauffe 48 h à 140°C. On alcalinise ensuite la solution par addition d'une solution 2N d'hydroxyde de sodium et on extrait avec de l'éther. L'extrait éthéré est lavé avec de l'eau, séché sur du sulfate de sodium anhydre et évaporé à sec.

Le résidu obtenu (1,9 g) est dissous dans un peu d'un mélange toluène-diéthylamine 9/1 et fixé sur une colonne contenant 500 g de silice. On élue ensuite avec un mélange toluène-diéthylamine 9/1, sous une pression de 4 bars. On isole ainsi 0,71 g du produit de départ et 0,68 g de méthoxy-6{[vinyl-3-(S) pipéridyl-4(R)]-3 propyl-1}-4 quinoléine sous forme d'une huile. Cette huile est mise en solution dans le méthanol et le produit ci-dessus est transformé en son chlorhydrate par addition d'une solution 8N d'acide chlorhydrique dans le méthanol.

Caractéristiques du chlorhydrate de méthoxy-6{[vinyl-3(S) pipéridyl-4(R)]-3 propyl-1}-4 quinoléine:

—Point de fusion: 151°C

—pouvoir rotatoire (mesuré dans l'eau à 25°C):

$$[\alpha]^{25}_{D} = -31°$$

—spectre de R.M.N. (solvant: deutérochloroforme; référence: tétraméthylsilane):

$$\delta_{10} : 5{,}4 \text{ ppm}$$

$$\delta_{11,\ 11'} : 5 \text{ ppm}$$

La méthoxy-6{[vinyl-3(R) pipéridyl-4(R)]-3 propyl-1}-4 quinoléine, produit de départ, peut être préparée comme suit:

A une suspension de 48 g de quinicine dans 200 ml de diéthylène-glycol et 23 g d'une solution aqueuse à 85% d'hydrate d'hydrazine, on ajoute 18 g d'hydroxyde de sodium en pastilles. On chauffe lentement et lorsqu'on atteint 110°C le milieu est homogène. On chauffe ensuite pendant 1 h à 130°C puis pendant 2 h à 150°C jusqu'à cessation de dégagement d'azote.

Le milieu réactionnel est jeté dans 1 l d'eau glacée. Une huile relargue qui est extraite par 500 ml d'éther. La phase organique est décantée, lavée, séchée sur sulfate de magnésium puis évaporée. On obtient ainsi une huile qui est constituée par la méthoxy-6{[vinyl-3(R) pipéridyl-4(R)]-3 propyl-1}-4 quinoléine.

Exemple 4

*Préparation de l'ester éthylique de l'acide [vinyl-3(S) pipéridyl-4(S)]acétique.*

11,98 g d'ester éthylique de l'acide [vinyl-3(R) pipéridyl-4(S)] acétique (ester éthylique de méroquinène) sont dissous dans 60 ml d'un mélange eau-éthanol (50/50). La solution est amenée à pH = 3,5 par addition d'une solution N d'acide chlorhydrique. Le mélange est introduit dans un autoclave en acier inoxydable de 225 ml et chauffé 32 h à 140°C. La solution hydroalcoolique est évaporée à sec. Le résidu obtenu (10,1 g) est dissous dans un peu d'un mélange toluène-diéthylamine 9/1 et fixé sur une colonne contenant 1 000 g de silice. On élue ensuite avec un mélange toluène-diéthylamine 9/1, sous une pression de 3 bars. On isole ainsi 2,9 g de produit de départ et 4,5 d'ester éthylique de l'acide[vinyl-3(S) pipéridyl-4(S)]acétique sous forme d'une huile.

Caractéristiques de l'ester éthylique de l'acide [vinyl-3(S) pipéridyl-4(S)]acétique:
—pouvoir rotatoire (mesuré dans le chloroforme à 25°C):

$$[\alpha]^{25}_{D} = -35,5°$$

—spectre de R.M.N. (solvant: deutérochloroforme; référence: tétraméthylsilane):

$$\delta_{10} = 5,4 \text{ ppm}$$

$$\delta_{11,\,11'} = 5 \text{ ppm}$$

L'ester éthylique de méroquinène, produit de départ, peut être préparé comme indiqué par R. LUKES [Chem. Listy. 47, 858 (1953)].

Exemple 5

*Préparation de la (méthoxy-6 quinolyl-4)-1 [vinyl-3(R) pipéridyl-4(R)]-3 propanone-1 (quinicine).*

On part de 3 g de chlorhydrate de (méthoxy-6 quinolyl-4)-1 [vinyl-3(S) pipéridyl-4(R)]-3 propanone-1, préparé comme indiqué à l'exemple 1. A partir de ce chlorhydrate on régénère la base libre. On ajoute à cette dernière 20 ml d'eau distillée et amène le pH à 2, par addition d'une solution N d'acide sulfurique. Le mélange est introduit dans un autoclave en acier inox de 225 ml et on chauffe 40 h à 140°C. On alcalinise ensuite la solution par addition d'une solution 2N d'hydroxyde de sodium et extrait avec de l'éther. L'extrait éthéré est lavé avec de l'eau, séché sur du sulfate de sodium anhydre et évaporé à sec.

Le résidu obtenu (2,22 g), dissous dans un peu d'un mélange toluène-diéthylamine 9/1, est fixé sur une colonne contenant 1 000 g de silice. On élue ensuite avec un mélange toluène-diéthylamine 9/1, sous une pression de 4 bars. On isole ainsi 1,03 g de produit de départ et 0,25 g de quinicine. Cette dernière, mise en solution dans le méthanol, est transformé en son chlorhydrate par addition d'une solution 8N d'acide chlorhydrique dans le méthanol.
caractéristiques du chlorhydrate de quinicine:
—point de fusion: 183°C
—pouvoir rotatoire (mesuré dans l'eau à 25°C):

$$[\alpha]^{25}_{D} = +44°$$

—spectre R.M.N. (solvant: deutérochloroforme; référence: tétraméthylsilane):

$$\delta_{10} : 6,48 \text{ ppm}$$

$$\delta_{11} : 5,21 \text{ ppm}$$

$$\alpha_{11'} : 5,18 \text{ ppm}$$

Exemple 6

*Préparation de la méthoxy-6{[vinyl-3(S) pipéridyl-4(R)]-3 propyl-1}-4-quinoléine.*

0,3 g de dichlorhydrate de méthoxy-6{[vinyl-3(R) pipéridyl-4(R)]-3 propyl-1}-4 quinoléine sont dissous dans 30 ml d'eau distillée. On ajuste le pH à 8 par addition d'une solution 1N d'hydroxyde de sodium. Ce mélange, introduit dans un autoclave en acier inoxydable de 100 ml, est chauffé à 160°C pendant 24 h. Après refroidissement, on amène pH 10 par addition d'une solution 2N d'hydroxyde de sodium et on extrait avec du dichlorométhane. La phase organique est lavée à l'eau, séchée sur du sulfate de magnésium et évaporée à sec.

Le résidu obtenu (0,22 g) contient 75% de méthoxy-6{[vinyl-3(S) pipéridyl-4(R)]-3 propyl-1}-4 quinoléine, qu'on isole par chromatographie liquide haute pression (éluant: mélange toluène-diéthylamine 9/1) et qu'on transforme en chlorhydrate en opérant comme indiqué à l'exemple 3. Point de fusion du chlorhydrate: 151°C.

Exemple 7

*Préparation de la méthoxy-6{[vinyl;-3(S) pipéridyl-4(R)]-3 propyl-1}-4 quinoléine.*

0,3 g de dichlorhydrate de méthoxy-6{[vinyl-3(R) pipéridyl-4(R)]-3 propyl-1}-4 quinoléine sont dissous dans 31 ml d'eau distillée. On ajoute 83 µl d'une solution aqueuse de formol à 37% en poids et on maintient le mélange pendant 20 heures à 70°C.

Après refroidissement, on alcalinise le mélange réactionnel par addition d'une solution 2N d'hydroxyde de sodium. L'huile qui relargue est extraite par 50 ml de dichlorométhane. La phase organique est lavée à l'eau, séchée sur du sulfate de magnésium puis évaporée.

On obtient 0,2 g d'un mélange contenant 88% de méthoxy-6 {[vinyl-3(S) pipéridyl-4(R)]-3 propyl-1}-4-quinoléine, qu'on isole et transforme en chlorhydrate comme indiqué à l'exemple 6. Point de fusion du chlorhydrate: 151 °C.

Exemple 8

*Préparation de la (méthoxy-6 quinolyl-4)-1 [vinyl-3(S) pipéridyl-4(R)]-3 propanone-1 (épiquinicine).*

926 mg de chlorhydrate de (méthoxy-6 quinolyl-4)-1 [vinyl-3(R) pipéridyl-4(R)]-3 propanone-1 (chlorhydrate de quinicine) sont dissous dans 100 ml d'eau distillée. Le pH de cette solution est amené à 8,9 par addition d'une solution N/10 d'hydroxyde de sodium. On prélève 30 ml de cette solution que l'on introduit dans un autoclave en acier inoxydable de 225 ml et on chauffe 24 h à 140°C. On alcalinise ensuite la solution par addition d'une solution 2N d'hyroxyde de sodium et on extrait avec de l'acétate d'éthyle. L'extrait organique est lavé avec de l'eau, séché sur du sulfate de sodium anhydre et évaporé à sec.

Le résidu obtenu (675 mg) est dissous dans un mélange toluène-diéthylamine 9/1 et fixé sur une colonne contenant 500 g de silice. On élue ensuite avec un mélange toluène-diéthylamine 9/1 sous une pression de 4 bars. On isole ainsi 183 mg de (méthoxy-6 quinolyl-4)-1 [vinyl-3(S) pipéridyl-4(R)]-3 propanone-1.

Ce dernier composé est mis en solution dans le méthanol et transformé en chlorhydrate par addition d'une solution 8N d'acide chlorhydrique dans le méthanol.

Caractéristiques du chlorhydrate de (méthoxy-6 quinolyl-4)-1 [vinyl-3(S) pipéridyl-4(R)]-3 propanone-1.

—point de fusion: 171 °C

—pouvoir rotatoire (mesuré dans l'eau à 25°C):

$$[\alpha]^{25}_{D} = -33{,}3°$$

—spectre de R.M.N. (solvant: deutérochloroforme; référence: tétraméthylsilane)

$$\delta_{10} = 5{,}6 \text{ ppm}$$

$$\delta_{11,\ 11'} = 5{,}1 \text{ ppm}$$

Exemple 9

*Préparation de la (méthoxy-6 quinolyl-4)-1 [vinyl-3(S) pipéridyl-4(R)]-3 propanone-1 (épiquinicine)*

925 mg de chlorhydrate de(méthoxy-6 quinolyl-4)-1[vinyl-3(R) pipéridyl-4(R)]-3 propanone-1 sont dissous dans 100 ml d'un mélange eau-éthanol 50/50, auxquels on ajoute 0,29 ml d'une solution aqueuse de formol à 37%. On prélève 30 ml de cette solution, que l'on amène à pH 4 par addition d'acide chlorhydrique N/10. On introduit la solution dans un autoclave en acier inoxydable de 225 ml. On chauffe 24 h à 70°C. On alcalinise ensuite la solution par addition d'une solution 2N d'hydroxyde de sodium, et on extrait avec de l'éther. L'extrait éthéré est lavé avec de l'eau, séché sur sulfate de sodium anhydre et évaporé à sec. Les résidu obtenu est dissous dans un mélange toluène-diéthylamine 9/1 et fixé sur une colonne contenant 500 g de silice. On élue ensuite avec un mélange toluène-diéthylamine 9/1 sous une pression de 4 bars. On isole ainsi 540 mg de (méthoxy-6 quinolyl-4)-1[vinyl-3(S) pipéridyl-4(R)]-3 propanone-1 qu'on transforme en chlorhydrate conformément au mode opératoire décrit à l'exemple 8. Les caractéristiques du chlorhydrate d'épiquinicine ainsi isolé sont identiques à celles trouvées à l'exemple 8.

**Revendications**

1. Procédé de transformation des composés de formule:

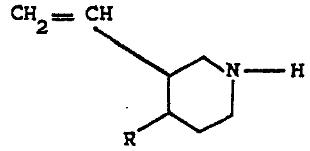

(I)

dans laquelle R est un atome d'hydrogène, un groupe alkyle ayant 1 à 4 atomes de carbone, un groupe phényle non substitué ou substitué par un atome de chlore, de brome ou de fluor par un groupe alkyle ayant 1 à 4 atomes de carbone, alcoxy ayant 1 à 4 atomes de carbone ou trifluorométhyle, ou le groupe $-(CH_2)_n-X-Z$ dans lequel n est 1 ou 2, X est un groupe méthylène ou carbonyle et Z représente un groupe alcoxy ayant 1 à 4 atomes de carbone, un reste aromatique ayant 6 à 10 atomes de carbone, éventuellement substitué par un atome de chlore, de brome ou de fluor ou par un groupe alkyle ayant 1 à 4 atomes de carbone, alcoxy ayant 1 à 4 atomes de carbone ou trifluorométhyle, ou un reste quinolyle-4 ou indolyle-2, éventuellement substitué par un atome de chlore, de brome ou de fluor ou par un groupe alkyle ayant 1 à 4 atomes de carbone ou alcoxy ayant 1 à 4 atomes de carbone, et pour

lesquels le carbone porteur du groupe vinyle possède la configuration rectus en les composés correspondants de formule (I) pour lesquels le carbone porteur du groupe vinyle possède la configuration sinister, et vice versa, caractérisé en ce que l'on chauffe, au sein d'un solvant protique ou d'un mélange de solvants protiques, en présence de formaldéhyde et à une température supérieure à 50°C et inférieure ou égale à 160°C ou en l'absence de formaldéhyde et à une température située dans la zone 120°C—160°C, un composé de formule (I) pour lequel le carbone porteur du groupe vinyle a la configuration rectus ou sinister, partiellement ou totalement salifié, et isole le composé de formule (I) pour lequel le carbone porteur du groupe vinyle a la configuration sinister ou rectus ainsi formé.

2. Procédé selon la revendication 1, caractérisé en ce que le chauffage est effectué dans un milieu aqueux ou un milieu mixte eau + alcool dont le pH est inférieur à 9, en l'absence de formaldéhyde.

3. Procédé selon la revendication 2, caractérisé en ce que le pH du milieu est entre 1 et 4.

4. Procédé selon la revendication 1, caractérisé en ce que le chauffage est effectué à une température dans la zone 50°C—80°C, dans un milieu aqueux ou dans un milieu mixte eau + alcool dont le pH est inférieur à 9, en présence de formaldéhyde.

5. Procédé selon l'une quelconque des revendications 1 à 4, caractérisé en ce que le composé de départ utilisé répond à la formule:

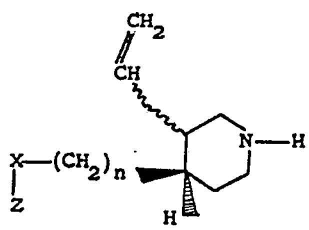

(II)

dans laquelle n, X et Z ont les significations indiquées à la revendication 1.

6. Procédé selon la revendication 5, caractérisé en ce que, dans le composé de formule (II), Z est un reste quinolyle-4 ou indolyle-2, non substitué ou substitué par un atome de chlore, de brome ou de fluor ou par un groupe alkyle ayant 1 à 4 atomes de carbone ou alcoxy ayant 1 à 4 atomes de carbone.

**Claims**

1. Process for the conversion of compounds of the formula:

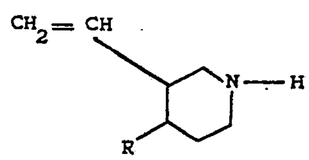

(I)

in which R is a hydrogen atom, an alkyl group having 1 to 4 carbon atoms, an unsubstituted phenyl group or a phenyl group substituted by a chlorine, bromine or fluorine atom or by an alkyl group having 1 to 4 carbon atoms, an alkoxy group having 1 to 4 carbon atoms or a trifluoromethyl group, or the group $-(CH_2)_n-X-Z$ in which n is 1 or 2, X is a methylene or carbonyl group and Z represents an alkoxy group having 1 to 4 carbon atoms, an aromatic residue having 6 to 10 carbon atoms, possibly substituted by a chlorine, bromine or fluorine atom or by an alkyl group having 1 to 4 carbon atoms, an alkoxy group having 1 to 4 carbon atoms or a trifluoremethyl group, or a 4-quinolyl or 2-indolyl residue possibly substituted by a chlorine, bromine or fluorine atom or by an alkyl group having 1 to 4 carbon atoms or an alkoxy group having 1 to 4 carbon atoms, and for which the carbon carrying the vinyl group possesses the rectus configuration in the corresponding compounds of formula (I) for which the carbon carrying the vinyl group possesses the sinister configuration, and vice versa, characterised in that a compound of formula (I) for which the carbon carrying the vinyl group has the rectus or sinister configuration, partially or wholly in salt form, is heated in a protic solvent or a mixture of protic solvents, in the presence of formaldehyde and at a temperature greater than 50°C and less than or equal to 160°C or in the absence of formaldehyde and at a temperature within the range 120°C—160°C, and the compound of formula (I) for which the carbon carrying the vinyl group has the sinister or rectus configuration thus formed is isolated.

2. Process according to claim 1, characterised in that the heating is effected in an aqueous medium or in a mixed water + alcohol medium of which the pH is less than 9, in the absence of formaldehyde.

3. Process according to claim 2, characterised in that the pH of the medium is between 1 and 4.

4. Process according to claim 1, characterised in that the heating is effected at a temperature in the range 50°C—80°C, in an aqueous medium or in a mixed water + alcool medium of which the pH is less than 9, in the presence of formaldehyde.

5. Process according to any one of claims 1 to 4, chacacterised in that the starting compound used corresponds to the formula:

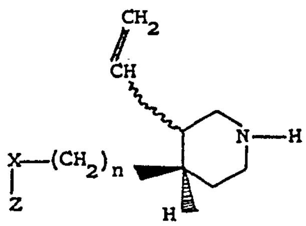

(II)

in which n, X and Z have the significance indicated in claim 1.

6. Process according to claim 5, characterised in that, in the compound of formula (II), Z is a 4-quinolyl or 2-indolyl residue, unsubstituted or substituted by a chlorine, bromine or fluorine atom or by an alkyl group having 1 to 4 carbon atoms or an alkoxy group having 1 to 4 carbon atoms.

**Patentansprüche**

1. Verfahren zur Umwandlung von Verbindungen der allgemeinen Formel I

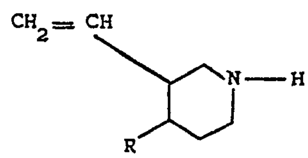

(I)

in der R ein Wasserstoffatom, eine Alkylgruppe mit 1 bis 4 Kohlenstoffatomen, eine unsubstituierte Phenylgruppe, eine durch ein Chloratom, ein Bromatom, ein Fluoratom, eine Alkylgruppe mit 1 bis 4 Kohlenstoffatomen, eine Alkoxygruppe mit 1 bis 4 Kohlenstoffatomen oder eine Trifluoromethylgruppe substituierte Phenylgruppe oder die Gruppe der Formel $-(CH_2)_n-X-Z$ bedeutet, in der n den Wert 1 oder 2 besitzt, X eine Methylengruppe oder eine Carbonylgruppe darstellt und Z für eine Alkoxygruppe mit 1 bis 4 Kohlenstoffatomen, eine aromatischen Rest mit 6 bis 10 Kohlenstoffatomen, der gegebenenfalls durch ein Chloratom, ein Bromatom, ein Fluoratom, eine Alkylgruppe mit 1 bis 4 Kohlenstoffatomen, eine Alkoxygruppe mit 1 bis 4 Kohlenstoffatomen oder eine Trifluormethylgruppe substituiert ist, oder einen 4-Chinolyl- oder 2-Indolyl-Rest, der gegebenenfalls durch ein Chloratom, eine Bromatom, ein Fluoratom, eine Alkylgruppe mit 1 bis 4 Kohlenstoffatomen oder eine Alkoxygruppe mit 1 bis 4 Kohlenstoffatomen substituiert ist, steht, bei denen das die Vinylgruppe tragende Kohlenstoffatom in der Rectus-Konfiguration vorliegt, in die entsprechenden Verbindungen der allgemeinen Formel I, bei denen das die Vinylgruppe tragende Kohlenstoffatom die Sinister-Konfiguration aufweist, und umgekehrt, dadurch gekennzeichnet, daß man eine teilweise oder vollständig in die Salzform überführte Verbindung der allgemeinen Formel I, in der das die Vinylgruppe tragende Kohlenstoffatom in der Rectus-Konfiguration oder der Sinister-Konfiguration vorliegt, in einem protischen Lösungsmittle oder einer Mischung von protischen Lösungsmitteln in Gegenwart von Formaldehyd auf eine Temperatur von mehr als 50°C und gleich oder kleiner 160°C oder in Abwesenheit von Formaldehyd auf eine Temperatur im Bereich von 120 bis 160°C erhitzt und die in dieser Weise gebildete Verbindung der allgemeinen Formel I, in der das die Vinylgruppe tragende Kohlenstoffatom in der Sinister-Konfiguration oder der Rectus-Konfiguration vorliegt, isoliert.

2. Verfahren nach Anspruch 1, dadurch gekennzeichnet, daß man das Erhitzen in Abwesenheit von Formaldehyd in einem wäßrigen Medium oder einem wäßrig-alkoholischen Medium mit einem pH-Wert von weniger als 9 bewirkt.

3. Verfahren nach Anspruch 2, dadurch gekennzeichnet, daß der pH-Wert des Mediums zwischen 1 und 4 liegt.

4. Verfahren nach Anspruch 1, dadurch gekennzeichnet, daß man das Erhitzen in Gegenwart von Formaldehyd bei einer Temperatur im Bereich von 50 bis 80°C in einem wäßrigen oder wäßrig-alkoholischen Medium, dessen pH-Wert weniger also 9 beträgt, bewirkt.

5. Verfahren nach einem der Ansprüche 1 bis 4, dadurch gekennzeichnet, daß die als Ausgangsmaterial eingesetzte Verbindung der nachstehenden Formel

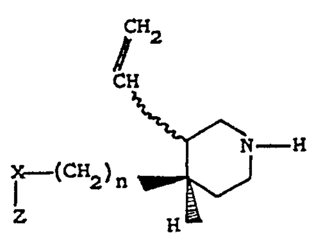

(II)

entspricht, in der n, X und Z die in Anspruch 1 angegebenen Bedeutungen besitzen.

6. Verfahren nach Anspruch 5, dadurch gekennzeichnet, daß bei der Verbindung der allgemeinen Formel II Z einen 4-Chinolyl- oder 2-Indolyl-Rest bedeutet, der nicht substituiert ist oder durch ein Chloratom, ein Bromatom, ein Fluoratom, eine Alkylgruppe mit 1 bis 4 Kohlenstoffatomen oder eine Alkoxygruppe mit 1 bis 4 Kohlenstoffatomen substituiert ist.